(19) **Europäisches Patentamt / European Patent Office / Office européen des brevets**

(11) **EP 2 902 772 B1**

(12) **EUROPEAN PATENT SPECIFICATION**

(45) Date of publication and mention
of the grant of the patent:
**17.10.2018 Bulletin 2018/42**

(21) Application number: **13839320.2**

(22) Date of filing: **05.04.2013**

(51) Int Cl.:
***G01N 21/64*** (2006.01)     ***G01N 27/447*** (2006.01)

(86) International application number:
**PCT/JP2013/002371**

(87) International publication number:
**WO 2014/045481 (27.03.2014 Gazette 2014/13)**

(54) **SPECTROSCOPIC ANALYSIS DEVICE, SPECTROSCOPIC ANALYSIS METHOD, AND COMPUTER-READABLE MEDIUM**

SPEKTROSKOPISCHE ANALYSEVORRICHTUNG, SPEKTROSKOPISCHES ANALYSEVERFAHREN UND COMPUTERLESBARES MEDIUM

DISPOSITIF AINSI QUE PROCÉDÉ D'ANALYSE SPECTROSCOPIQUE, ET SUPPORT LISIBLE PAR ORDINATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.09.2012 JP 2012206023**

(43) Date of publication of application:
**05.08.2015 Bulletin 2015/32**

(73) Proprietor: **NEC Corporation**
**Tokyo 108-8001 (JP)**

(72) Inventor: **ASOGAWA, Minoru**
**Tokyo 108-8001 (JP)**

(74) Representative: **Betten & Resch**
**Patent- und Rechtsanwälte PartGmbB**
**Maximiliansplatz 14**
**80333 München (DE)**

(56) References cited:
JP-A- 2005 274 496     JP-A- 2011 053 074
JP-B2- 3 727 031     US-A1- 2008 212 866
US-A1- 2011 117 127     US-B1- 6 333 501
US-B1- 6 738 502     US-B1- 6 863 791

• WITTWER C T ET AL: "Real-time multiplex PCR assays", METHODS, ACADEMIC PRESS, US, vol. 25, no. 4, December 2001 (2001-12), pages 430-442, XP002265718, ISSN: 1046-2023, DOI: 10.1006/METH.2001.1265
• O.N.VYLEGZHANIN ET AL.: 'CALCULATION OF THE CONCENTRATIONS OF THE COMPONENTS OF A MIXTURE BASED ON ITS SPECTRUM USING PSEUDOINVERSION' JOURNAL OF APPLIED SPECTROSCOPY vol. 52, no. 3, 1990, pages 275 - 279, XP008179154
• SASAKI A ET AL: "Monitoring intracellular degradation of exogenous DNA using diffusion properties", JOURNAL OF CONTROLLED RELEASE, ELSEVIER, AMSTERDAM, NL, vol. 143, no. 1, 2 April 2010 (2010-04-02) , pages 104-111, XP026964328, ISSN: 0168-3659 [retrieved on 2010-03-12]

EP 2 902 772 B1

## Description

## Technical Field

The present invention relates to a spectroscopic analysis apparatus, a spectroscopic analysis method, and a program, and more particularly, to a spectroscopic analysis apparatus, a spectroscopic analysis method, and a program that perform an analysis using spectra obtained by dispersing light generated in a sample.

## Background Art

An apparatus for specifying a gene locus is disclosed (Patent literature 1). Patent literature 1 discloses using capillary electrophoresis. Patent literature 1 further discloses performing labeling using fluorescence. Patent literature 1 further discloses using Raman spectrometry.

## Citation List

## Patent Literature

**Patent literature 1:** Published Japanese Translation of PCT International Publication for Patent Application, No. 2005-527904

## Summary of Invention

## Technical Problem

Patent literature 1 discloses a method for performing an analysis using a computer program. There are some cases, however, in which samples cannot be appropriately analyzed by the method disclosed in Patent literature 1. The use of inverse generalized matrix is generally known for determining the concentration of molecules such as DNA associated with a fluorescent label (see C.T Wittwer et Al. Methods, Academic Press, Vol.25 (4), December 2001, p.430-442, DOI:10.1006/METH.2001.1265; US6863791; US6738502; US2008/0212866).

The present invention aims to provide a spectroscopic analysis apparatus, a spectroscopic analysis method, and a program capable of appropriately analyzing the concentration of the bases in DNA fragments.

## Solution to Problem

The invention is defined in the claims. A spectroscopic analysis apparatus according to one exemplary aspect includes: a light source that generates light incident on a sample including a plurality of substances labeled by a plurality of labeled substances; a spectrometer that disperses observed light generated in the sample by the light incident on the sample; a detector that detects the observed light dispersed by the spectrometer to output observed spectral data; and a processor that analyzes the plurality of substances included in the sample based on the observed spectral data output from the detector, the processor analyzing the substances included in the sample from the observed spectral data using a generalized inverse of a matrix including, as an element, reference spectrum data set for the plurality of labeled substances.

A spectroscopic analysis method according to one exemplary aspect of the present invention includes: irradiating a sample including a plurality of substances labeled by a plurality of labeled substances with light; dispersing observed light generated in the sample by the light incident on the sample; detecting the observed light that is dispersed to output observed spectral data; obtaining a generalized inverse of a matrix having, as an element, reference spectrum data set for the plurality of labeled substances; and analyzing the substances included in the sample using the generalized inverse and the observed spectral data.

A program according to one exemplary aspect of the present invention is a program for causing a computer to execute a spectroscopic analysis method that analyzes a sample using observed spectral data obtained by performing spectrometry for light generated in the sample, in which: the spectroscopic analysis method obtains a generalized inverse of a matrix of reference spectrum data using, as a matrix, the reference spectrum data set for a plurality of labeled substances that label the plurality of substances included in the sample, and the spectroscopic analysis method analyzes the substances included in the sample using the observed spectral data and the generalized inverse.

## Advantageous Effects of Invention

According to the present invention, it is possible to provide a spectroscopic analysis apparatus, a spectroscopic analysis method, and a program capable of appropriately analyzing the base concentration in DNA fragments.

## Brief Description of Drawings

Fig. 1 is a diagram schematically showing a configuration of a spectroscopic analysis apparatus according to an exemplary embodiment of the present invention;

Fig. 2 is a graph showing spectra of fluorescence generated from fluorescent substances that label DNA:

Fig. 3 is a graph showing spectra of the fluorescent substance and generalized inverse data;

Fig. 4 is a diagram showing a matrix calculation expression for performing DNA analysis;

Fig. 5 is a diagram showing a matrix calculation expression for performing DNA analysis; and

Fig. 6 is a diagram showing a calculation expression for performing DNA analysis.

**Description of Embodiments**

With reference to the accompanying drawings, an exemplary embodiment of the present invention will be described. The exemplary embodiment described below is an example of the present invention and the present invention is not limited to the following exemplary embodiment. Throughout the specification and the drawings, the same components are denoted by the same reference symbols.

In this exemplary embodiment, a DNA sequence analysis is performed using a plurality of fluorescent substances having different emission wavelengths. Specifically, DNA is extracted from human cells. DNA fragments are amplified by a polymerase chain reaction (PCR) and are labeled by the fluorescent substances. The fluorescent substance may be, for example, 5-FAM, JOE, NED, and ROX. As a matter of course, the fluorescent substance used for the labeling is not particularly limited. In this example, a plurality of fluorescent substances having different peak wavelengths are used for the labeling. Different bases are labeled by different fluorescent substances.

Different PCR products labeled by fluorescence are supplied to a capillary and are electrophoresed in gel. In a state in which a voltage is applied by electrophoresis, the migration velocity varies depending on the size of the DNA fragments. The migration distance increases with decreasing number of bases. It is therefore possible to separate the DNA fragments by size. When PCR products in the capillary are irradiated with excitation light emitted from a light source, fluorescence is generated from fluorescent substances. The fluorescence generated from the fluorescent substances is spectroscopically measured to obtain observed spectral data. The observed spectral data is obtained for each size of the DNA fragments. By analyzing these observed spectral data, it is possible to quantify DNA of a particular sequence and to execute DNA testing.

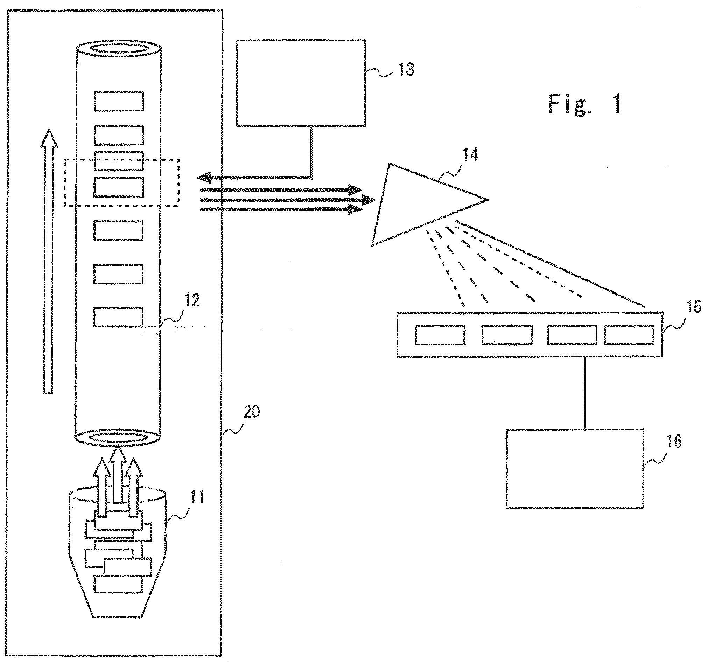

With reference to Fig. 1, the spectroscopic analysis apparatus according to the present invention will be described. Fig. 1 is a diagram showing a configuration of the spectroscopic analysis apparatus. The spectroscopic analysis apparatus includes an injection part 11, a capillary 12, a light source 13, a spectrometer 14, a detector 15, a processor 16, a microchip 20, and an optical fiber 31. In this example, an analysis is performed using capillary electrophoresis.

PCR products including DNA fragments labeled by fluorescent substances are injected into the injection part 11. In this example, the DNA fragments which are the sample are labeled by a plurality of fluorescent substances. For example, fluorescent substances such as 5-FAM, JOE, NED, and ROX are used depending on the base sequence of the DNA fragments. As a matter of course, the type and the number of the fluorescent substances used for the labeling are not particularly limited.

The injection part 11 is communicated with the capillary 12 on the microchip 20. Electrodes (not shown) are arranged on both ends of the capillary 12 provided in the microchip 20 and a voltage is applied to the electrodes. The capillary 12 and the injection part 11 are filled with an electrophoresis medium such as agarose gel. Accordingly, since the electrophoretic velocity becomes low according to the number of bases of the DNA fragments, the DNA fragments are separated by size.

The light source 13 irradiates the medium in the capillary with light. The light source 13 may be, for example, an argon ion laser light source that emits excitation light having a wavelength of 488 nm or 514.5 nm.

The light emitted from the light source 13 is incident on the capillary 12. In this example, 8-lane capillaries 12 are provided in parallel in the microchip 20. When the 8-lane capillaries 12 are irradiated with excitation light, the fluorescent substances that label the DNA fragments in the capillary 12 generate fluorescence. The fluorescence generated by the fluorescent substances is observed light.

The fluorescence generated by the fluorescent substances in the sample is input to the spectrometer 14. The spectrometer 14 includes, for example, a prism or diffraction grating, and disperses the fluorescence. In summary, the fluorescence is spatially dispersed according to the wavelength. The fluorescence spatially dispersed by the spectrometer 14 is input to the detector 15. Accordingly, the fluorescence generated by the fluorescent substances becomes observed light observed by the detector.

The detector 15 is, for example, a photodetector such as a CCD device, and includes light-receiving elements arranged along a dispersion direction. Accordingly, fluorescence having different wavelengths is detected for each of the light-receiving elements arranged in the dispersion direction. The detector 15 detects the spectra of the fluorescent substances that have labeled the DNA fragments and outputs the observed spectral data to the processor 16. For example, the spectrum having a wavelength region of 640 to 860 nm is detected by the spectrometer 14 and the detector 15. As a matter of course, the wavelength region that can be spectroscopically measured by the spectrometer 14 and the detector 15 is not particularly limited. The wavelength region can be appropriately set according to the excitation light wavelength or the fluorescent substance used as a label.

The detector 15 outputs to the processor 16 the light intensity in each wavelength that can be observed as observed spectral data. The number of pieces of data included in the observed spectral data varies according to the dispersion performance or the like of the spectrometer 14.

The processor 16 is an information processing device such as a personal computer, and performs processing according to a control program. Specifically, the processor 16 stores an analysis program that analyzes the observed spectral data output from the detector 15. The processor 16 executes processing according to the analysis program. The processor 16 analyzes the plurality of substances included in the sample based on the observed spectral data output from the detector 15. The concentration of the DNA fragments is thus obtained. It is therefore possible to perform DNA testing.

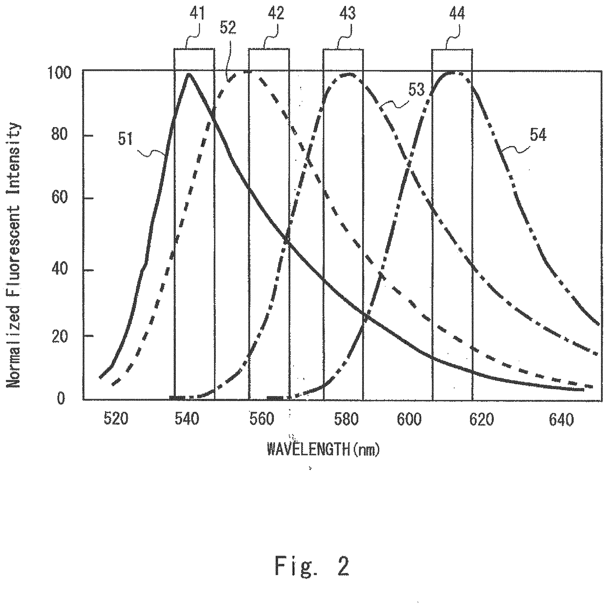

The processing in the processor 16 is one of the characteristics of the spectroscopic analysis method according to this exemplary embodiment. In the following description, the processing in the processor 16 will be described. Fig. 2 is a diagram schematically showing spectra of the fluorescent substances that have labeled the DNA fragments. In this description, a case in which the DNA fragments are labeled using four fluorescent substances of 5-FAM, JOE, NED, and ROX will be described.

In Fig. 2, the fluorescent spectrum when 5-FAM is irradiated with the excitation light is a reference spectrum 51. In a similar way, the fluorescent spectrum when JOE is irradiated with the excitation light is a reference spectrum 52, the fluorescent spectrum when NED is irradiated with the excitation light is a reference spectrum 53, and the fluorescent spectrum when ROX is irradiated with the excitation light is a reference spectrum 54. The wavelength of the excitation light is 488 nm. In Fig. 2, the horizontal axis represents the wavelength and the vertical axis is the fluorescent intensity normalized to the peak intensity which is set at 100.

The reference spectra 51 to 54 of the fluorescent substances are known and are different depending on the fluorescent substance. In short, the reference spectra have different peak wavelengths. For example, the reference spectrum 51 of 5-FAM has a peak wavelength of about 540 nm, the reference spectrum 52 of JOE has a peak wavelength of about 560 nm, the reference spectrum 53 of NED has a peak wavelength of about 580 nm, and the reference spectrum 54 of ROX has a peak wavelength of about 610 nm.

The observed spectrum detected by the detector 15 is obtained by overlapping the reference spectra 51-54 shown in Fig. 2 according to the concentration of the fluorescent substances. By analyzing the observed spectral data to obtain the concentration of each fluorescent substance, the distribution of the concentration of each base can be obtained.

When the concentration of the fluorescent substances included in the sample is obtained, windows 41 to 44 each having a predetermined wavelength width are normally set. The window 41 is set to a value close to the peak wavelength of the reference spectrum 51 of 5-FAM, the window 42 is set to a value close to the peak wavelength of the reference spectrum 52 of JOE, the window 43 is set to a value close to the peak wavelength of the reference spectrum 53 of NED, and the window 44 is set to a value close to the peak wavelength of the reference spectrum 54 of ROX. The light intensity data of the observed spectral data is accumulated for each of the windows 41 to 44.

The concentration of the fluorescent substances is obtained from the integrated value of each of the windows 41 to 44. For example, the concentration of 5-FAM, JOE, NED, and ROX are respectively set to b, g, y, and r. Further, the integrated values of the windows 41 to 44 are respectively set to $I_{540}$, $I_{560}$, $I_{580}$, and $I_{610}$. By solving the simultaneous equations with four unknowns shown in the following Expression (1) for b, g, y, and r, the concentration of the fluorescent substances is obtained.

$$I_{540} = b x_b + g y_b + y b_b + r w_b$$
$$I_{560} = b x_g + g y_g + y b_g + r w_g$$
$$I_{580} = b x_y + g y_y + y b_y + r w_y$$
$$I_{610} = b x_r + g y_r + y b_r + r w_r \qquad (1)$$

Here, the integrated values of the windows 41 to 44 in the reference spectrum 51 are respectively denoted by coefficients $x_b$, $y_b$, $bb$, and $w_b$. In a similar way, the integrated values of the windows 41 to 44 in the reference spectrum 52 are respectively denoted by coefficients $x_g$, $y_g$, $b_g$, and $w_g$, the integrated values of the windows 41 to 44 in the reference spectrum 53 are respectively denoted by coefficients $x_y$, $y_y$, $b_y$, and $w_y$, and the integrated values of the windows 41 to 44 in the reference spectrum 54 are respectively denoted by coefficients $x_r$, $y_r$, $b_r$, and $w_r$. Since the reference spectra 51 to 54 of each fluorescent substance are known, these coefficients are all known. Accordingly, the processor 16 solves the above simultaneous equations for b, g, y, and r, whereby it is possible to obtain the concentration of the fluorescent substances.

When the windows 41 to 44 according to the peak wavelength of the fluorescent spectrum are set as described above, however, the analysis may not be appropriately performed. For example, it may be difficult to set the windows 41 to 44 according to the peak wavelength of the fluorescent spectrum. When the width of the windows 41 to 44 is narrow, for example, the number of pieces of information to be accumulated becomes small and the noise increases. This is because noise normally decreases proportional to the square root of the number to be accumulated. In summary, while it is advantageous to make the width of the windows 41 to 44 wider in terms of S/N, data of another fluorescent substance is included if the windows 41 to 44 are too wide. It is therefore difficult to set appropriate windows 41 to 44.

However, it is possible to make an appropriate analysis by using the spectroscopic analysis method according to this exemplary embodiment. In order to simplify the following description, a case in which the sample is labeled by two fluorescent substances will be described.

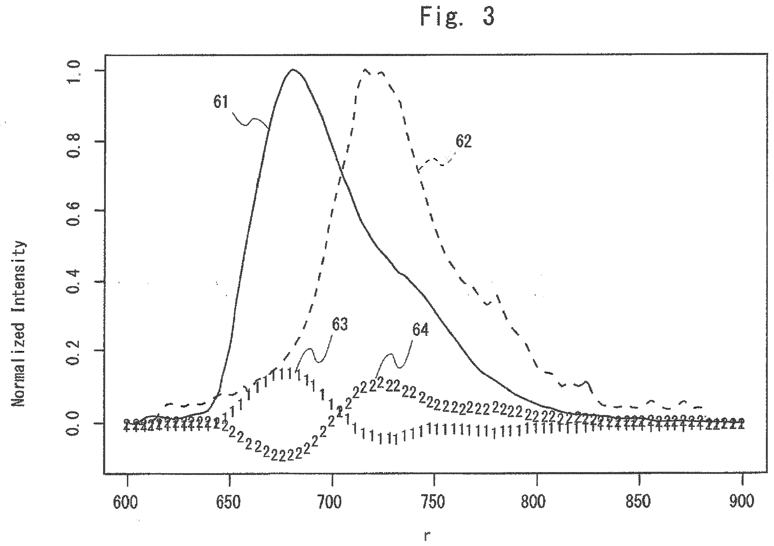

It is assumed that two fluorescent substances include reference spectra 61 and 62 as shown in Fig. 3. These are reference spectra used to obtain the concentration of the fluorescent substances and are known. The reference spectra 61 and 62 differ for each fluorescent substance. In Fig. 3, the intensity is normalized so that the peak intensity of the reference spectra 61 and 62 becomes 1.

The processor 16 calculates the generalized inverse of a matrix having, as an element, light intensity data of the reference spectra 61 and 62 set for the plurality of labeled substances. The data of the generalized inverse is shown as generalized inverse data 63 and 64 in the graph shown in Fig. 3. The processor 16 analyzes the DNA fragments included in the sample from the observed spectral data. In the following description, the matrix calculation performed by the processor 16 to analyze the sample will be described.

The matrix of the light intensity data in each wavelength included in the observed spectral data is denoted by b. When the observed spectral data includes, m (m is an integer larger than 2) pieces of light intensity data, for example, the matrix b has m rows and one column. The elements included in the matrix b are denoted by b1, b2,...bm.

Further, the matrix of the light intensity data included in the reference spectra 61 and 62 of the two fluorescent substances is denoted by A. The matrix A has m rows and two columns. The elements of the matrix A are m pieces of light intensity data $A_{11}, A_{21}, A_{31},...A_{m1}$ included in the reference spectrum 61 and m pieces of light intensity data $A_{12}, A_{22}, A_{32},...A_{m2}$ included in the reference spectrum 62. The light intensity data $A_{11}, A_{21}, A_{31},...A_{m1}$ are the elements of the first row and the light intensity data $A_{12}, A_{22}, A_{32},...A_{m2}$ are the elements of the second row. Since the number of fluorescent substances that label the sample is 2, the matrix A has m rows and two columns. The number of rows of the matrix A increases in accordance with the increase in the number of fluorescent substances to be used. When the sample is labeled by four fluorescent substances corresponding to four bases, for example, the matrix A has m rows and four columns.

Note that the number of pieces of light intensity data of the reference spectra 61 and 62 is the same as the number of pieces of light intensity data included in the observed spectrum. In summary, the wavelength where the light intensity data is present is the same in the observed spectrum and the reference spectra 61 and 62. As a matter of course, when the number of pieces of data of the reference spectra 61 and 62 is different from the number of pieces of observed spectrum data, the number of pieces of data may be made the same by complementing data.

Further, the matrix of the concentration of the fluorescent substances included in the sample is denoted by x. Since the number of fluorescent substances used for the labeling is two, the matrix x has two rows and one column. The elements included in the matrix x are denoted by $x_1$ and $x_2$. The processor 16 executes processing for obtaining the matrix x.

In each wavelength, the following Expression (2) is established.

$$bj = Aj1 \times x1 + Aj2 \times x2 \quad ...(2)$$

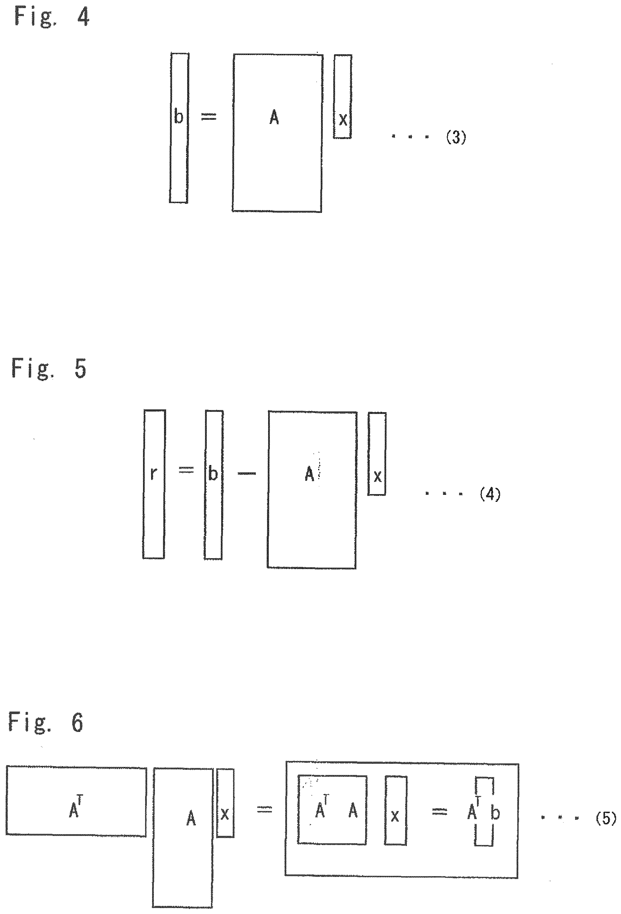

Note that j is any integer from 1 to m. From the product of the concentration of the fluorescent substances used for the labeling and the light intensity data of the reference spectrum in one wavelength, the light intensity data of the observed spectrum in this wavelength can be calculated. Since Expression (2) is established for any desired wavelength, when Expression (2) is expressed using the matrix A, the matrix b, and the matrix x, Expression (3) in Fig. 4 can be obtained.

In an ideal measurement, Expression (3) in Fig. 4 is established. While there are two elements $x_1$ and $x_2$ of the matrix x to be obtained, the number of conditional expressions is m. Since m is larger than 2, the number of conditions is too large. In order to solve this problem, the approximate solution that minimizes the error r shown in Expression (4) in Fig. 5 is obtained. This approximate solution is the least squares problem that minimizes |r|.

Since A is not a square matrix, there is no inverse matrix. It is also possible, however, to calculate a generalized inverse (or generalized inverse matrix). By using the generalized inverse, x can be calculated from Expression (3) shown in Fig. 4. In summary, the processor 16 obtains the least squares optimal solution by the generalized inverse matrix.

It is assumed that the matrix is $A^T$= two rows and m columns. As shown in Expression (5) in Fig. 6, $A^TA$ is a square matrix (in this example, two rows and two columns), whereby it is possible to obtain the inverse matrix. When the inverse matrix of $A^TA$ is $(A^TA)^{-1}$, the matrix x can be calculated by the following Expression (6) from Expression (5) in Fig. 6.

$$x = (A^T A)^{-1} A^T b \quad ...(6)$$

Expression (6) means obtaining the least squares solution that minimizes the error r shown in Expression (4) in Fig. 5. Since the matrix A includes the known reference spectra 61 and 62, it is possible to unambiguously calculate $(A^TA)^{-1}A^T$.

It is possible to calculate the matrix x by multiplying the matrix b of the observed spectrum by $(A^TA)^{-1}A^T$. It is therefore possible to obtain the concentration of the fluorescent substances. When $C=(A^TA)^{-1}A^T$, for example, C is the generalized inverse. The product of the generalized inverse C of A and the matrix b is then obtained. The element of the generalized

inverse $(A^TA)^{-1}A^T$ is generalized inverse data 63 and 64 shown in Fig. 3. In summary, the matrix has two rows and m columns with the generalized inverse data 63 in the first row and the generalized inverse data 64 in the second row.

It is therefore possible to calculate the concentration of the plurality of fluorescent substances used for the labeling in a simple way. Further, since the windows 41 to 44 are not set as shown in Fig. 2, it is possible to calculate the concentration with higher accuracy. For example, by setting the windows 41 to 44, light intensity data of the observed spectrum outside the windows 41 to 44 is not used. In summary, the number of pieces of light intensity data to obtain the concentration of the fluorescent substances becomes small, which causes degradation of the accuracy of the calculation. Meanwhile, in this exemplary embodiment, a larger number of pieces of light intensity data included in the observed spectrum can be used, whereby it is possible to decrease the noise and to improve the measurement accuracy. It is therefore possible to obtain an accurate calculation of the concentration and to perform a more appropriate analysis.

As described above, the processor 16 analyzes the plurality of substances included in the sample based on the observed spectral data output from the detector 15. Accordingly, the processor 16 obtains the generalized inverse of the matrix of the data of the reference spectra 61 and 62 using, as a matrix, the data of the reference spectra 61 and 62 set for the plurality of labeled substances that label the plurality of substances. The processor 16 analyzes the substances included in the sample using the observed spectral data and the generalized inverse. If the generalized inverse of the matrix of the reference spectrum is calculated in advance, the processing can be executed in a shorter period of time.

It is therefore possible to perform an analysis using a larger number of observed spectral data. It is therefore possible to appropriately analyze the sample based on the spectrum of the fluorescence and to perform DNA testing with a small measurement error.

As described above, by electrophoresing the PCR amplified sample, the DNA fragments are separated by size. The DNA fragments in the capillary are irradiated with light to detect the observed spectrum in each size of the DNA fragments. The plurality of observed spectra are subjected to the above processing to calculate the concentration of each base. The distribution of the concentration of the bases is obtained for each size of the DNA fragments. The DNA testing is carried out according to the base sequence of the DNA fragment. It is therefore possible to perform DNA testing with higher accuracy.

The control for analyzing the above sample may be executed by a computer program. The control program described above can be stored and provided to a computer using any type of non-transitory computer readable media. Non-transitory computer readable media include any type of tangible storage media. Examples of non-transitory computer readable media include magnetic storage media (such as flexible disks, magnetic tapes, hard disk drives, etc.), optical magnetic storage media (e.g. magneto-optical disks), CD-ROM (Read Only Memory), CD-R, CD-R/W, and semiconductor memories (such as mask ROM, PROM (Programmable ROM), EPROM (Erasable PROM), flash ROM, RAM (Random Access Memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

Further, the exemplary embodiment of the present invention includes not only the case in which the functions of the above exemplary embodiment are achieved by the computer executing the program that achieves the functions of the above exemplary embodiment but also a case in which this program achieves the functions of the above exemplary embodiment in collaboration with an application software or an operating system (OS) operated on the computer.

While the present invention has been described with reference to the exemplary embodiment, the present invention is not limited to the above exemplary embodiment. Various changes that can be understood by those skilled in the art may be made on the configuration and the details of the present invention within the scope of the present invention.

This application is based upon and claims the benefit of priority from Japanese Patent Application No. 2012-206023, filed on September 19, 2012.

**Reference Signs List**

| | |
|---|---|
| 11 | INJECTION PART |
| 12 | CAPILLARY |
| 13 | LIGHT SOURCE |
| 14 | SPECTROMETER |
| 15 | DETECTOR |
| 16 | PROCESSOR |
| 20 | CHIP |
| 41-44 | WINDOWS |
| 51-54 | REFERENCE SPECTRA |
| 61, | 62 REFERENCE SPECTRA |
| 63, 63 | GENERALIZED INVERSE DATA |

**Claims**

1. A spectroscopic analysis apparatus comprising:

a light source (13) that is adapted to generate light incident on a sample comprising DNA fragments labeled by a plurality of fluorescent labeled substances in accordance with a base sequence, the different bases being labeled by different fluorescent substances;

a spectrometer (14) that is adapted to disperse observed light generated in the sample by the light incident on the sample;

a detector (15) that is adapted to detect the observed light dispersed by the spectrometer to output observed spectral data; and

a processor (16) that is adapted to analyze the DNA fragments included in the sample based on the observed spectral data output from the detector, the processor analyzing a concentration of each base of the DNA fragment from the observed spectral data using a generalized inverse of a matrix including, as an element, reference spectrum data set for the plurality of labeled substances, wherein the sample that is PCR amplified is subjected to electrophoresis to separate the DNA fragments by size, the spectral data is obtained for each size of the DNA fragments, **characterized in that** the concentration of the each base of the DNA fragments separated by size is analyzed based on a plurality of the observed spectral data and the generalized inverse of the matrix, a distribution of the concentration of the base is obtained for each size of the DNA fragments by analyzing the concentration of the each base of the DNA fragments separated by size, and a DNA test is performed according to the distribution of the concentrations of the base.

2. The spectroscopic analysis apparatus according to Claim 1, **characterized in that** the spectroscopic analysis apparatus is adapted to calculate the product of the matrix of the observed spectral data and the generalized inverse to calculate a ratio of the plurality of labeled substances included in the sample.

3. The spectroscopic analysis apparatus according to Claim 1 or 2, **characterized in that** the labeled substances are fluorescent substances and a reference spectrum is set based on a known fluorescent spectrum of the fluorescent substances.

4. A spectroscopic analysis method comprising:

irradiating a sample comprising DNA fragments labeled by a plurality of fluorescent labeled substances with light in accordance with a base sequence, the different bases being labeled by different fluorescent substances;

dispersing observed light generated in the sample by the light incident on the sample;

detecting the observed light that is dispersed to output observed spectral data;

obtaining a generalized inverse of a matrix having, as an element, reference spectrum data set for the plurality of labeled substances; and

analyzing the DNA fragments and a concentration of each base of the DNA fragment included in the sample using the generalized inverse of the matrix and the observed spectral data, wherein the sample that is PCR amplified is subjected to electrophoresis to separate the DNA fragments by size,

the spectral data is obtained for each size of the DNA fragments, **characterized in that** the concentration of the each base of the DNA fragments separated by size is analyzed based on a plurality of the observed spectral data and the generalized inverse of the matrix,

a distribution of the concentration of the base is obtained for each size of the DNA fragments by analyzing the concentration of the each base of the DNA fragments separated by size, and

a DNA test is performed according to the distribution of the concentrations of the base.

5. The spectroscopic analysis method according to Claim 4, **characterized in that** it comprises calculating the product of the matrix of the observed spectral data and the generalized inverse to calculate a ratio of the plurality of labeled substances included in the sample.

6. The spectroscopic analysis method according to Claim 4 or 5, **characterized in that** the labeled substances are fluorescent substances and a known fluorescent spectrum of the fluorescent substances is set as a reference spectrum.

7. A non-transitory computer readable medium storing a program for causing a computer to execute a spectroscopic analysis method that analyzes a sample comprising DNA fragments labeled by a plurality of fluorescent labeled substances in accordance with a base sequence, the different bases being labeled by different fluorescent substances, using observed spectral data obtained by performing spectrometry for light generated in the sample, wherein the spectroscopic analysis method obtains a generalized inverse of a matrix of reference spectrum data using, as a matrix, the reference spectrum data set for a plurality of labeled substances that label the plurality of substances included in the sample, and the spectroscopic analysis method analyzes the DNA fragments **characterized in that** the spectroscopic analysis method analyzes a concentration of each base of the DNA fragment included in the sample using the observed spectral data and the generalized inverse of the matrix, wherein the sample that is PCR amplified is subjected to electrophoresis to separate the DNA fragments by size,

the spectral data is obtained for each size of the DNA fragments,

the concentration of the each base of the DNA fragments separated by size is analyzed based on a plurality of the observed spectral data and the generalized inverse of the matrix, a distribution of the concentration of the base is obtained for each size of the DNA fragments by analyzing the concentration of the each base of the DNA fragments separated by size, and

a DNA test is performed according to the distribution of the concentrations of the base.

8. The non-transitory computer readable medium according to Claim 7, **characterized in that** it comprises calculating the product of the matrix of the observed spectral data and the generalized inverse to calculate a ratio of the plurality of labeled substances included in the sample.

9. The non-transitory computer readable medium according to Claim 7 or 8, **characterized in that** the labeled substances are fluorescent substances and a known fluorescent spectrum of the fluorescent substances is set as a reference spectrum.

**Patentansprüche**

1. Spektroskopisches Analysegerät, umfassend:

eine Lichtquelle (13), die dazu ausgelegt ist, Licht zu generieren, das auf eine Probe fällt, die DNA-Fragmente umfasst, die mit einer Mehrzahl von fluoreszierenden markierten Substanzen gemäß einer Basensequenz markiert sind, wobei die verschiedenen Basen mit verschiedenen fluoreszierenden Substanzen markiert sind;
ein Spektrometer (14), das dazu ausgelegt ist, beobachtetes Licht zu dispergieren, das in der Probe durch das auf die Probe fallende Licht generiert ist;
einen Detektor (15), der dazu ausgelegt ist, das beobachtete Licht zu detektieren, das durch das Spektrometer dispergiert wird, um beobachtete Spektraldaten auszugeben; und
einen Prozessor (16), der dazu ausgelegt ist, die in der Probe enthaltenen DNA-Fragmente basierend auf den von dem Detektor ausgegebenen Spektraldaten zu analysieren, wobei der Prozessor eine Konzentration von jeder Base des DNA-Fragments aus den beobachteten Spektraldaten unter Verwendung einer generalisierten Inversen einer Matrix analysiert, die als ein Element Referenz-Spektrumsdaten enthält, die für die Mehrzahl von markierten Substanzen eingestellt sind,
wobei

die Probe, die PCR-verstärkt ist, einer Elektrophorese unterzogen wird, um die DNA-Fragmente nach Größe zu separieren,
wobei die Spektraldaten für jede Größe der DNA-Fragmente erhalten werden,
**dadurch gekennzeichnet, dass**
die Konzentration jeder Base der DNA-Fragmente, nach Größe separiert, analysiert wird basierend auf einer Mehrzahl der beobachteten Spektraldaten und der generalisierten Inversen der Matrix,
eine Verteilung der Konzentration der Base für jede Größe der DNA-Fragmente erhalten wird durch Analysieren der Konzentration jeder Base der DNA-Fragmente, nach Größe separiert, und
ein DNA-Test gemäß der Verteilung der Konzentrationen der Base durchgeführt wird.

2. Spektroskopisches Analysegerät nach Anspruch 1, **dadurch gekennzeichnet, dass** das spektroskopische Analysegerät dazu ausgelegt ist, das Produkt der Matrix der beobachteten Spektraldaten und der generalisierten Inversen zu berechnen, um ein Verhältnis der Mehrzahl von markierten Substanzen zu berechnen, die in der Probe enthalten sind.

3. Spektroskopisches Analysegerät nach Anspruch 1 oder 2, **dadurch gekennzeichnet, dass** die markierten Substanzen fluoreszierende Substanzen sind, und ein Referenz-Spektrum eingestellt ist basierend auf einem bekannten Fluoreszenzspektrum der fluoreszierenden Substanzen.

4. Spektroskopisches Analyseverfahren, umfassend:

Bestrahlen einer Probe, die DNA-Fragmente enthält, die mit einer Mehrzahl von fluoreszierenden Markierungssubstanzen markiert sind, mit Licht, gemäß einer Basensequenz, wobei die verschiedenen Basen mit verschiedenen fluoreszierenden Substanzen markiert sind;
Dispergieren von beobachteten Licht, das in der Probe durch das Licht generiert wird, das auf die Probe fällt;
Detektieren des beobachteten Lichts, das dispergiert wird, um beobachtete Spektraldaten auszugeben;
Erhalten einer generalisierten Inversen einer Matrix, die als ein Element Referenz-Spektrumsdaten hat, die für die Mehrzahl von markierten Substanzen eingestellt sind; und
Analysieren der DNA-Fragmente und einer Konzentration jeder Base der DNA-Fragmente, die in der Probe enthalten sind, unter Verwendung der generalisierten Inversen der Matrix und der beobachteten Spektraldaten,
wobei die Probe, die PCR-verstärkt ist, einer Elektrophorese unterzogen wird, um die DNA-Fragmente nach Größe zu separieren,
wobei die Spektraldaten für jede Größe der DNA-Fragmente erhalten werden,
**dadurch gekennzeichnet, dass** die Konzentration jeder Base der DNA-Fragmente, nach Größe separiert, analysiert wird basierend auf einer Mehrzahl der beobachteten Spektraldaten und der generalisierten Inversen der Matrix, eine Verteilung der Konzentration der Base für jede Größe der DNA-Fragmente erhalten wird durch Analysieren der Konzentration jeder Base der DNA-Fragmente, separiert nach Größe, und ein DNA-Test gemäß der Verteilung der Konzentrationen der Base durchgeführt wird.

5. Spektroskopisches Analyseverfahren nach Anspruch 4, **dadurch gekennzeichnet, dass** es ein Berechnen des Produkts der Matrix der beobachteten Spektraldaten und der generalisierten Inversen umfasst, um ein Verhältnis der Mehrzahl von markierten Substanzen zu berechnen, die in der Probe enthalten sind.

6. Spektroskopisches Analyseverfahren nach Anspruch 4 oder 5, **dadurch gekennzeichnet, dass** die markierten Substanzen fluoreszierende Substanzen sind, und ein bekanntes Fluoreszenzspektrum der fluoreszierenden Substanzen als ein Referenz-Spektrum eingestellt wird.

7. Nichtflüchtiges computerlesbares Medium, auf dem ein Programm gespeichert ist, um einen Computer zu veranlassen, ein spektroskopisches Analyseverfahren auszuführen, das eine Probe analysiert, die DNA-Fragmente enthält, die mit einer Mehrzahl von fluoreszierenden Markierungssubstanzen markiert sind , gemäß einer Basensequenz, wobei die verschiedenen Basen mit verschiedenen fluoreszierenden Substanzen markiert sind, unter Verwendung von beobachteten Spektraldaten, die durch Durchführen einer Spektrometrie für Licht erhalten werden, das in der Probe generiert wird, wobei das spektroskopische Analyseverfahren eine generalisierte Inverse einer Matrix von Referenz-Spektrumsdaten erhält unter Verwendung, als eine Matrix, der Referenz Spektrumsdaten, die für eine Mehrzahl von markierten Substanzen eingestellt sind, die die Mehrzahl von Substanzen markieren, die in der Probe enthalten sind, und wobei das spektroskopische Analyseverfahren die DNA-Fragmente analysiert, **dadurch gekennzeichnet, dass** das spektroskopische Analyseverfahren eine Konzentration jeder Base des DNA-Fragments analysiert, das in der Probe enthalten ist, unter Verwendung der beobachteten Spektraldaten und der generalisierten Inversen der Matrix, wobei die Probe, die PCR-verstärkt ist, einer Elektrophorese unterzogen wird, um die DNA-Fragmente nach Größe zu separieren,
wobei die Spektraldaten für jede Größe der DNA-Fragmente erhalten werden, wobei die Konzentration jeder Base der DNA-Fragmente, nach Größe separiert, analysiert wird basierend auf einer Mehrzahl der beobachteten Spektraldaten und der generalisierten Inversen der Matrix, wobei eine Verteilung der Konzentration der Base für jede Größe der DNA-Fragmente erhalten wird durch Analysieren der Konzentration jeder Base der DNA-Fragmente, nach Größe separiert, und wobei ein DNA-Test gemäß der Verteilung der Konzentrationen der Base durchgeführt wird.

8. Nichtflüchtiges computerlesbares Medium nach Anspruch 7,**dadurch gekennzeichnet, dass** es ein Berechnen des Produkts der Matrix der beobachteten Spektraldaten und der generalisierten Inversen umfasst, um ein Verhältnis der Mehrzahl von markierten Substanzen zu berechnen, die in der Probe enthalten sind.

9. Nichtflüchtiges computerlesbares Medium nach Anspruch 7 oder 8, **dadurch gekennzeichnet, dass** die markierten Substanzen fluoreszierende Substanzen sind, und ein bekanntes Fluoreszenzspektrum der fluoreszierenden Substanzen als eine Referenz-Spektrum eingestellt ist.

**Revendications**

1. Appareil d'analyse spectroscopique comprenant :

une source de lumière (13) qui est adaptée pour générer une lumière incidente sur un échantillon contenant des fragments d'ADN étiquetés par une pluralité de substances fluorescentes étiquetées selon une séquence de base, les différentes bases étant étiquetées par différentes substances fluorescentes ;
un spectromètre (14) qui est adapté pour disperser une lumière observée générée dans l'échantillon par la lumière incidente sur l'échantillon ;
un détecteur (15) qui est adapté pour détecter la lumière observée dispersée par le spectromètre pour délivrer en sortie des données spectrales observées ; et
un processeur (16) qui est adapté pour analyser les fragments d'ADN inclus dans l'échantillon sur la base des données spectrales observées délivrées en sortie à partir du détecteur, le processeur analysant une concentration de chaque base du fragment d'ADN à partir des données spectrales observées en utilisant un inverse généralisé d'une matrice incluant, en tant qu'un élément, des données spectrales de référence définies pour la pluralité de substances étiquetées,

dans lequel

l'échantillon qui est amplifié PCR est soumis à électrophorèse afin de séparer les fragments d'ADN par taille,

les données spectrales sont obtenues pour chaque taille des fragments d'ADN,

**caractérisé en ce que** la concentration de la chaque base des fragments d'ADN séparés par taille est analysée sur la base d'une pluralité des données spectrales observées et de l'inverse généralisé de la matrice,

une distribution de la concentration de la base est obtenue pour chaque taille des fragments d'ADN en analysant la concentration de la chaque base des fragments d'ADN séparés par taille, et

un test ADN est exécuté selon la distribution des concentrations de la base.

2. Appareil d'analyse spectroscopique selon la revendication 1, **caractérisé en ce que** l'appareil d'analyse spectroscopique est adapté pour calculer le produit de la matrice des données spectrales observées et de l'inverse généralisé afin de calculer un rapport de la pluralité de substances étiquetées incluses dans l'échantillon.

3. Appareil d'analyse spectroscopique selon la revendication 1 ou 2, **caractérisé en ce que** les substances étiquetées sont des substances fluorescentes et un spectre de référence est défini sur la base d'un spectre fluorescent connu des substances fluorescentes.

4. Procédé d'analyse spectroscopique comprenant les étapes :

irradier un échantillon contenant des fragments d'ADN étiquetés par une pluralité de substances fluorescentes étiquetées avec de la lumière selon une séquence de base, les différentes bases étant étiquetées par différentes substances fluorescentes ;

disperser la lumière observée générée dans l'échantillon par la lumière incidente sur l'échantillon ;

détecter la lumière observée qui est dispersée afin de délivrer en sortie des données spectrales observées ;

obtenir un inverse généralisé d'une matrice ayant, en tant qu'un élément, des données spectrales de référence définies pour la pluralité de substances étiquetées ; et

analyser les fragments d'ADN et d'une concentration de chaque base du fragment d'ADN inclus dans l'échantillon en utilisant l'inverse généralisé de la matrice et des données spectrales observées,

dans lequel

l'échantillon qui est amplifié PCR est soumis à électrophorèse afin de séparer les fragments d'ADN par taille,

les données spectrales sont obtenues pour chaque taille des fragments d'ADN,

**caractérisé en ce que** la concentration de la chaque base des fragments d'ADN séparés par taille est analysée sur la base d'une pluralité des données spectrales observées et de l'inverse généralisé de la matrice,

une distribution de la concentration de la base est obtenue pour chaque taille des fragments d'ADN en analysant la concentration de la chaque base des fragments d'ADN séparés par taille, et

un test ADN est exécuté selon la distribution des concentrations de la base.

5. Procédé d'analyse spectroscopique selon la revendication 4, **caractérisé en ce qu'**il comprend le calcul du produit de la matrice des données spectrales observées et de l'inverse généralisé afin de calculer un rapport de la pluralité de substances étiquetées incluses dans l'échantillon.

6. Procédé d'analyse spectroscopique selon la revendication 4 ou 5, **caractérisé en ce que** les substances étiquetées sont des substances fluorescentes et un spectre fluorescent connu des substances fluorescentes est défini en tant qu'un spectre de référence.

7. Support non transitoire lisible par ordinateur contenant un programme pour amener un ordinateur à exécuter un procédé d'analyse spectroscopique qui analyse un échantillon contenant des fragments d'ADN étiquetés par une pluralité de substances fluorescentes étiquetées selon une séquence de base, les différentes bases étant étiquetées par différentes substances fluorescentes, en utilisant des données spectrales observées obtenues en exécutant une spectrométrie pour une lumière générée dans l'échantillon,

dans lequel

le procédé d'analyse spectroscopique obtient un inverse généralisé d'une matrice de données spectrales de référence en utilisant, en tant qu'une matrice, les données spectrales de référence définies pour une pluralité de substances étiquetées qui étiquettent la pluralité de substances incluses dans l'échantillon, et

le procédé d'analyse spectroscopique analyse les fragments d'ADN,

**caractérisé en ce que** le procédé d'analyse spectroscopique analyse une concentration de chaque base du fragment d'ADN inclus dans l'échantillon en utilisant les données spectrales observées et l'inverse généralisé de la matrice,

dans lequel l'échantillon qui est amplifié PCR est soumis à électrophorèse afin de séparer les fragments d'ADN par taille,

les données spectrales sont obtenues pour chaque taille des fragments d'ADN,

la concentration de la chaque base des fragments d'ADN séparés par taille est analysée sur la base d'une pluralité des données spectrales observées et de l'inverse généralisé de la matrice,

une distribution de la concentration de la base est obtenue pour chaque taille des fragments d'ADN en analysant la concentration de la chaque base des fragments d'ADN séparés par taille, et

un test ADN est exécuté selon la distribution des concentrations de la base.

8. Support non transitoire lisible par ordinateur selon la revendication 7, **caractérisé en ce qu'**il comprend le calcul du produit de la matrice des données spectrales observées et de l'inverse généralisé afin de calculer un rapport de la pluralité de substances étiquetées incluses dans l'échantillon.

9. Support non transitoire lisible par ordinateur selon la revendication 7 ou 8, **caractérisé en ce que** les substances étiquetées sont des substances fluorescentes et un spectre fluorescent connu des substances fluorescentes est défini en tant qu'un spectre de référence.

Fig. 1

EP 2 902 772 B1

Fig. 2

13

Fig. 3

Fig. 4

$$b = A \, x \qquad \cdots \ (3)$$

Fig. 5

$$r = b - A \, x \qquad \cdots \ (4)$$

Fig. 6

$$A^T \, A \, x = A^T A \, x = A^T b \qquad \cdots \ (5)$$

## REFERENCES CITED IN THE DESCRIPTION

**Patent documents cited in the description**

- JP 2005527904 W
- US 6863791 B
- US 6738502 B
- US 20080212866 A
- JP 2012206023 A

**Non-patent literature cited in the description**

- **C.T WITTWER et al.** Methods. Academic Press, December 2001, vol. 25, 430-442